# EUROPÄISCHE PATENTSCHRIFT

(11) **EP 4 295 787 B1**
(45) Veröffentlichungstag und Bekanntmachung des Hinweises auf die Patenterteilung: **12.03.2025**
(21) Anmeldenummer: 23179088.2
(22) Anmeldetag: 14.06.2023
(51) Int. Cl.: A61B 17/15, A61F 2/46, A61F 2/38

(54) **CHIRURGISCHE VORRICHTUNG**
SURGICAL DEVICE
DISPOSITIF CHIRURGICAL

(30) Priorität: 20.06.2022 DE 102022206117
(43) Veröffentlichungstag der Anmeldung: 27.12.2023
(73) Patentinhaber: Aesculap AG, 78532 Tuttlingen (DE)
(72) Erfinder: FIRMBACH, Franz-Peter, 78576 Emmingen-Liptingen (DE); STOFFELS, Lilli, 75365 Calw (DE)
(74) Vertreter: Patentanwälte Ruff, Wilhelm, Beier, Dauster & Partner mbB

(56) Entgegenhaltungen:
- DE-A1- 102020 110 346
- GB-A- 2 398 011
- US-A- 5 514 143

## Beschreibung

Die Erfindung betrifft eine chirurgische Vorrichtung mit den oberbegrifflichen Merkmalen des Anspruchs 1.

Bei einer Kniegelenkersatzoperation (englisch: Total Knee Arthroplasty (TKA)) werden abgenutzte oder durch Krankheit oder einen Unfall beeinträchtigte Gelenkflächen des Femurs und/oder der Tibia durch künstliche Gelenkflächen einer Kniegelenkprothese ersetzt. Solche Kniegelenkprothesen umfassen üblicherweise eine Femurkomponente und eine Tibiakomponente. Die Femurkomponente wird am distalen Ende des Femurs implantiert. Die Tibiakomponente wird am proximalen Ende der Tibia implantiert.

Vor der Implantation der prothetischen Komponenten werden der distale Femur und die proximale Tibia reseziert. Hierfür bringt der Chirurg unterschiedliche Resektionsschnitte an und trennt Knochen- und/oder Knorpelmaterial von dem jeweiligen Knochen ab. Durch die Resektion wird der jeweilige Knochen in seiner Form an die aufzunehmende prothetische Komponente angepasst.

Die Resektion kann auf der Grundlage unterschiedlicher Konzepte ausgeführt werden. Ein Konzept zielt darauf ab, die Spannungen der Bänder des Knies bei der Gelenkbewegung ausgeglichen zu halten. Hierdurch soll eine bessere Funktion der Kniegelenkprothese gewährleistet werden. Dieses Konzept wird allgemein als "gap balancing" bezeichnet. Bei anderen Konzepten entfernt der Operateur mittels der Resektion eine bestimmte Menge an Knochen- und/oder Knorpelmaterial. Solche Konzepte werden allgemein als "measured resection" bezeichnet. Die Ausrichtung der Resektionsschnitte in Bezug auf die Anatomie des Patienten bestimmt die spätere Ausrichtung der implantierten Komponenten und folglich auch die Orientierung der prothetischen Gelenkachsen. Die Ausrichtung der Resektionsschnitte ist daher von besonderer Bedeutung.

Bei der Ausrichtung der Resektionsschnitte werden in erster Linie drei Ansätze unterschieden: mechanisch, anatomisch und kinematisch. Bei der mechanischen Ausrichtung wird die proximale Tibia senkrecht zur Längsachse des Tibiaschafts reseziert. Die Resektion des distalen Femurs erfolgt hieran angepasst. Erforderlichenfalls werden Bandentlastungen (englisch: ligament releases) durchgeführt. Bei der anatomischen Ausrichtung wird versucht, die Tibia in einem Varus-Winkel von 3° zu resezieren. Die Femurresektion und Bandentlastungen werden mit dem Ziel einer geraden Hüft-Knie-Knöchel-Achse des Beins durchgeführt. Ziel der kinematischen Ausrichtung (englisch: kinematic alignment, nachfolgend abgekürzt als KA) ist es, die künstlichen Gelenkflächen der prothetischen Komponenten auf dem Niveau der präarthritischen, defektfreien natürlichen Gelenkflächen zu implantieren.

Bei einer KA erfolgt die Ausrichtung der Resektionsschnitte oftmals ausgehend von dem distalen Femur. Die Resektion der proximalen Tibia erfolgt hieran angepasst. In diesem Zusammenhang wird auch von einer Übertragung der Ausrichtungen und/oder Schnitte auf die Tibia gesprochen. Zu diesem Zweck sind spezielle chirurgische Instrumente bekannt, die auch als tibiales Ausricht- und/oder Übertragungswerkzeug (englisch: tibial cut alignment guide) bezeichnet werden. Solche Instrumente erlauben eine Übertragung der Ausrichtung der femoralen Resektionsschnitte auf die Tibia und weisen oftmals einen extramedullären Ausrichtstab auf (englisch: extramedullary alignment rod). Bei einer üblichen Vorgehensweise wird eine parallele Ausrichtung des extramedullären Ausrichtstabs zu der anterioren Kante der Tibia angestrebt.

Aus der US 5 514 143 A und der GB 2 398 011 A ist jeweils eine chirurgische Vorrichtung mit den oberbegrifflichen Merkmalen des Anspruchs 1 bekannt.

Weiter ist aus der DE 10 2020 110 346 A1 eine chirurgische Vorrichtung in Form einer Ausrichtungsvorrichtung für eine tibiale Resektionsführung bekannt. Die bekannte Vorrichtung weist eine Klemmvorrichtung, einen Sägeblock und eine Antastvorrichtung auf. Die Klemmvorrichtung kann als eine Art Stützgeometrie aufgefasst werden.

Aufgabe der Erfindung ist es, eine vereinfachte und besonders präzise parallele Ausrichtung des extramedullären Ausrichtstabs zu ermöglichen.

Diese Aufgabe wird durch das Bereitstellen einer chirurgischen Vorrichtung mit den Merkmalen des Anspruchs 1 gelöst.

Die erfindungsgemäße chirurgische Vorrichtung weist auf: einen Stützabschnitt mit wenigstens zwei Stützgeometrien, welche entlang einer proximodistal erstreckten Stützgeraden voneinander beabstandet und jeweils zum Abstützen auf einer anterioren Kante einer Tibia eingerichtet sind, und einen von dem Stützabschnitt anterior aufragenden Referenzabschnitt mit wenigstens zwei Referenzgeometrien, welche entlang einer proximodistal erstreckten Referenzgeraden voneinander beabstandet sind, wobei die Referenzgerade um einen anterioren Abstand von der Stützgeraden beabstandet und parallel zu der Stützgeraden erstreckt ist, wobei die wenigstens zwei Referenzgeometrien und/oder die Referenzgerade anstelle der anterioren Kante der Tibia als Referenz zum Ausrichten des extramedullären Ausrichtstabs dienen, und wobei die wenigstens zwei Referenzgeometrien jeweils zur wenigstens mediolateral formschlüssigen Lagerung des extramedullären Ausrichtstabs eingerichtet sind. Die erfindungsgemäße Vorrichtung erlaubt eine besonders präzise und für den ausführenden Operateur vereinfachte parallele Ausrichtung des extramedullären Ausrichtstabs. Die zwischen den wenigstens zwei Stützgeometrien proximodistal erstreckte Stützgerade liegt in der Verwendung auf der anterioren Kante der Tibia auf. Diese wird nachfolgend auch als Tibiavorderkante bezeichnet. Die wenigstens zwei Stützgeometrien sind bei unterschiedlichen Ausgestaltungen unterschiedlich ausgeführt und können beispielsweise "punktförmig", linienförmig oder flächenhaft sein. Die wenigstens zwei Stützgeometrien des Stützabschnitts sind in der Verwendung der chirurgischen Vorrichtung entlang der Tibiavorderkante voneinander beabstandet. Der Stützabschnitt kann grundsätzlich eine beliebige Form aufweisen. Entsprechendes gilt sinngemäß für den Referenzabschnitt. Die wenigstens zwei Referenzgeometrien des Referenzabschnitts sind in der Verwendung der chirurgischen Vorrichtung entlang der Tibiavorderkante voneinander beabstandet. Die Referenzgerade ist zwischen den wenigstens zwei Referenzgeometrien sowie anterior parallel versetzt zu der Stützgeraden - und damit auch der Tibiavorderkante - erstreckt. In Bezug auf eine Rückenlage des Patienten ist die Referenzgerade folglich um den (anterioren) Abstand oberhalb der Tibiavorderkante angeordnet. Die Referenzgerade und/oder die Referenzgeometrien dienen der eigentlichen Parallelausrichtung des extramedullären Ausrichtstabs. Durch die anteriore Beabstandung der Referenzgeraden und/oder der Referenzgeometrien liegt der extramedulläre Ausrichtstab anteroposterior näher an der Referenzgeraden und/oder den Referenzgeometrien als an der Tibiavorderkante. Durch den insoweit verringerten Abstand kann der ausführende Operateur einfacher und präziser feststellen, ob eine parallele Ausrichtung vorliegt oder nicht. Dies insbesondere dann, wenn der Patient eine starke Varus/Valgus-Stellung der Tibia aufweist. Auch die Einrichtung der wenigstens zwei Referenzgeometrien zur formschlüssigen Lagerung des extramedullären Ausrichtstabs trägt zur besonders einfachen und präzisen Parallelausrichtung des extramedullären Ausrichtstabs bei. Denn die formschlüssige Lagerung an den wenigstens zwei Referenzgeometrien wirkt einer ungewollten Relativverlagerung des extramedullären Ausrichtstabs entgegen. Hierdurch wird Fehlern bei der Parallelausrichtung entgegengewirkt. Die formschlüssige Lagerung wirkt wenigstens in mediolateraler Richtung. Zusätzlich kann ein Formschluss in anteroposteriorer Richtung vorgesehen sein. Vorzugsweise sind die wenigstens zwei Referenzgeometrien jeweils zur proximodistal gleitbeweglichen Lagerung des extramedullären Ausrichtstabs eingerichtet. Bei unterschiedlichen Ausgestaltungen sind die wenigstens zwei Referenzgeometrien unterschiedlich ausgeführt, beispielsweise jeweils als Vertiefung, Kerbe, Ausnehmung, Rille, Nut und/oder Bohrung. Der besagte Formschluss ist lösbar und kann beispielsweise in Form einer Rast-, Steck-, Klemm- und/oder Schnappverbindung ausgebildet sein. Bei einer Ausgestaltung ist die chirurgische Vorrichtung einteilig ausgeführt. Bei einer weiteren Ausgestaltung ist die chirurgische Vorrichtung mehrteilig aufgebaut. Bei einer Ausgestaltung sind der Referenzabschnitt und der Stützabschnitt einstückig zusammenhängend gefertigt. Bei einer weiteren Ausgestaltung sind die besagten Abschnitte als separate Bauteile gefertigt und miteinander zusammengefügt. Die chirurgische Vorrichtung ist vorzugsweise aus einem Kunststoffmaterial und/oder aus Metall gefertigt. Bei einer Ausgestaltung ist die chirurgische Vorrichtung eine Einwegvorrichtung zum einmaligen Gebrauch. Bei einer weiteren Ausgestaltung ist die chirurgische Vorrichtung eine Mehrwegvorrichtung zum mehrmaligen Gebrauch. Der extramedulläre Ausrichtstab ist nicht Bestandteil der chirurgischen Vorrichtung.

Die in dieser Beschreibung verwendeten Lage- und Richtungsbezeichnungen beziehen sich auf den Körper eines Patienten, insbesondere dessen Tibia, und sind insoweit gemäß ihrer üblichen anatomischen Bedeutung zu verstehen. Folglich bedeuten "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet. Weiter werden Bezeichnungen wie "Oberseite" in Bezug auf eine posterior gerichtete Blickrichtung verwendet. Dementgegen werden Bezeichnungen wie "Unterseite" in Bezug auf eine anterior gerichtete Blickrichtung verwendet.

Weiter gemäß der Erfindung weist der Referenzabschnitt wenigstens zwei erste Referenzgeometrien, welche entlang einer ersten Referenzgeraden voneinander beabstandet sind, und zwei zweite Referenzgeometrien, welche entlang einer zweiten Referenzgeraden voneinander beabstandet sind, auf, wobei die erste Referenzgerade in einem anterioren ersten Abstand von der Stützgeraden beabstandet ist, und wobei die zweite Referenzgerade in einem anterioren zweiten Abstand von der Stützgeraden beabstandet ist. Durch die unterschiedlichen Abstände der wenigstens zwei Referenzgeraden und/oder jeweils zugehörigen Referenzgeometrien kann die chirurgische Vorrichtung an unterschiedlich großen Knochenstrukturen verwendet werden. Mit anderen Worten ausgedrückt, ist die chirurgische Vorrichtung ohne weiteres bei Patienten unterschiedlicher Größe verwendbar. Es versteht sich, dass sowohl die erste Referenzgerade als auch die zweite Referenzgerade proximodistal und parallel zu der Stützgeraden erstreckt sind. In Bezug auf eine Rückenlage des Patienten sind die Referenzgeraden übereinander angeordnet. Vorzugsweise weist die chirurgische Vorrichtung eine Vielzahl von paarweise angeordneten Referenzgeometrien mit jeweils einer zugehörigen Referenzgeraden auf. Vorzugsweise sind mehr als fünf, bevorzugt mehr als zehn, besonders bevorzugt mehr als zwanzig Referenzgeraden vorhanden. Hierdurch kann der extramedulläre Ausrichtstab besonders präzise und fein abgestuft in unterschiedlichen Abständen parallel zu der Tibiavorderkante ausgerichtet werden.

In weiterer Ausgestaltung der Erfindung sind die Referenzgeometrien jeweils als Bohrung ausgebildet, so dass wenigstens zwei erste Bohrungen und zwei zweite Bohrungen vorhanden sind. Durch eine solche Gestaltung der Referenzgeometrien kann der extramedulläre Ausrichtstab besonders einfach und zuverlässig formschlüssig gelagert werden. Zudem ergibt sich eine einfache und kostengünstige Fertigung der Referenzgeometrien und damit auch der chirurgischen Vorrichtung. Die Bohrungen sind jeweils proximodistal und entlang ihrer zugehörigen Referenzgeraden längserstreckt. Die beiden ersten Bohrungen und die beiden zweiten Bohrungen bilden jeweils ein Bohrungspaar. In der Verwendung ist der extramedulläre Ausrichtstab an und/oder in einem Paar der Bohrungen formschlüssig lösbar gelagert, beispielsweise an und/oder in den beiden ersten Bohrungen oder den beiden zweiten Bohrungen. Die Lagerung ist wenigstens mediolateral formschlüssig. Zusätzlich kann ein, insbesondere lösbarer, Formschluss in anteroposteriorer Richtung vorhanden sein. Vorzugsweise ist der extramedullärer Ausrichtstab in Längsrichtung der Bohrungen gleitbeweglich. Vorzugsweise sind ein Außendurchmesser des extramedullären Ausrichtstabs und ein Innendurchmesser der Bohrungen derart aufeinander abgestimmt, dass eine spielfreie Lagerung erreicht ist. Vorzugsweise weisen die Bohrungen jeweils einen runden, bevorzugt kreisrunden, Querschnitt auf.

In weiterer Ausgestaltung der Erfindung sind die ersten Bohrungen und die zweiten Bohrungen anteroposterior miteinander verbunden, wodurch der extramedulläre Ausrichtstab ausgehend von einer Lagerung an den zwei ersten Bohrungen anteroposterior und/oder in seiner Radialrichtung verschiebbar ist in eine Lagerung an den zwei zweiten Bohrungen und umgekehrt. Hierdurch kann der extramedulläre Ausrichtstab zwischen unterschiedlichen anterioren Abständen verschoben werden, ohne dass er hierfür axial aus den ersten Bohrungen herausgezogen und hiernach in die zweiten Bohrungen eingeschoben werden müsste oder umgekehrt. Die anteroposteriore Verbindung zwischen den Bohrungen kann beispielsweise schlitzförmig ausgebildet sein. Alternativ oder zusätzlich können Bohrungen eine Bohrungs- oder auch Lochreihe bilden, wobei ein Abstand zwischen benachbarten Bohrungsmittelpunkten geringer ist als ein Durchmesser der Bohrungen. Bei einer Ausgestaltung sind die Bohrungen jeweils in Radialrichtung elastisch nachgiebig. Dies vereinfacht eine Verlagerung des extramedullären Ausrichtstabs zwischen benachbarten Bohrungen und/oder unterschiedlichen anterioren Abständen und/oder von einem der Bohrungspaare in ein anderes der Bohrungspaare. Bei einer weiteren Ausgestaltung weist der extramedulläre Ausrichtstab alternativ oder zusätzlich einen rotationsunsymmetrischen Querschnitt auf. Zur Verlagerung zwischen benachbarten Bohrungen kann der extramedulläre Ausrichtstab um seine Längsachse gedreht werden, so dass er durch die jeweilige Verbindung zwischen den benachbarten Bohrungen hindurchgeführt werden kann.

In weiterer Ausgestaltung der Erfindung ist der Referenzabschnitt im Bereich der Bohrungen elastisch formnachgiebig. Durch die elastische Formnachgiebigkeit kann der extramedulläre Ausrichtstab besonders einfach anteroposterior von einem der Bohrungspaare in ein anderes der Bohrungspaare bewegt werden. Die elastische Formnachgiebigkeit ist bei einer Ausgestaltung werkstoffbedingt. Beispielsweise kann der Referenzabschnitt wenigstens im Bereich der Bohrungen aus einem formnachgiebigen Kunststoff gefertigt sein. Bei einer weiteren Ausgestaltung ist die elastische Formnachgiebigkeit alternativ oder zusätzlich gestaltungsbedingt. Beispielsweise kann der Referenzabschnitt wenigstens im Bereich der Bohrungen dünnwandig gestaltet sein.

In weiterer Ausgestaltung der Erfindung ist der Referenzabschnitt als Bügel mit zwei Längsschenkeln und einem Querschenkel ausgebildet, wobei die zwei Längsschenkel proximodistal voneinander beabstandet sowie jeweils anteroposterior längserstreckt sind und die Referenzgeometrien aufweisen, und wobei der Querschenkel proximodistal zwischen den zwei Längsschenkeln längserstreckt ist und dieselben miteinander verbindet. Dies ist eine besonders bevorzugte Ausgestaltung der Erfindung. Sofern die Referenzgeometrien jeweils als Bohrung ausgeführt sind, sind diese jeweils proximodistal durch einen der Längsschenkel erstreckt. Vorzugsweise ist der Querschenkel einends der beiden Längsschenkel angeordnet und der Stützabschnitt ist andernends der Längsschenkel angeordnet und/oder ausgebildet. Vorzugsweise sind die Längsschenkel parallel zueinander längserstreckt. Vorzugsweise ist der Querschenkel parallel zu der Stützgeraden und/oder der Referenzgeraden/den Referenzgeraden längserstreckt. Der Querschenkel dient zum einen als Verbindungselement zwischen den beiden Längsschenkeln. Alternativ oder zusätzlich dient der Querschenkel als Handgriff zur einfachen Handhabung der chirurgischen Vorrichtung. Bei einer alternativen Ausgestaltung sind lediglich die zwei Längsschenkel, nicht jedoch der Querschenkel vorhanden.

In weiterer Ausgestaltung der Erfindung weisen die Längsschenkel jeweils eine Bohrungsreihe mit einer Vielzahl von anteroposterior aneinandergereihten Bohrungen als Referenzgeometrien auf. Dies ist eine besonders bevorzugte Ausgestaltung der Erfindung. Die Bohrungen der Bohrungsreihen sind vorzugsweise anteroposterior miteinander verbunden. Mit anderen Worten ausgedrückt, gehen die Bohrungen einer Bohrungsreihe vorzugsweise radial ineinander über.

In weiterer Ausgestaltung der Erfindung ist der Stützabschnitt durch Stirnenden der Längsschenkel gebildet, wobei jeweils ein Stirnende eine der Stützgeometrien aufweist. Bei dieser Ausgestaltung bilden die Stirnenden der Längsschenkel gleichsam Füße oder Fußabschnitte zum Abstützen auf der Tibiavorderkante. Hierdurch wird ein besonders einfacher Aufbau der chirurgischen Vorrichtung erreicht. Die Längsschenkel sind jeweils zwischen einem ersten Stirnende und einem zweiten Stirnende längserstreckt. Der Stützabschnitt wird beispielsweise durch die beiden ersten Stirnenden gebildet. Der Querschenkel, sofern vorhanden, ist in diesem Fall vorzugsweise zwischen den beiden zweiten Stirnenden längserstreckt.

In weiterer Ausgestaltung der Erfindung weisen die Stützgeometrien jeweils eine Gabelform mit einem ersten Astabschnitt und einem zweiten Astabschnitt auf. Mit anderen Worten ausgedrückt, sind die Stirnenden der Längsschenkel jeweils nach Art einer Astgabel mit einem ersten Astabschnitt und einem zweiten Astabschnitt gestaltet. Die Gabelform wirkt einem Abrutschen der chirurgischen Vorrichtung von der Tibiavorderkante entgegen.

In weiterer Ausgestaltung der Erfindung ist der Stützabschnitt als Platte mit einer posterior orientierten Unterseite und einer anterior orientierten Oberseite ausgebildet, wobei die Unterseite eben ist und die wenigstens zwei Stützgeometrien aufweist und/oder bildet, und wobei der Referenzabschnitt von der Oberseite aufragt. In der Verwendung wird die Platte mit ihrer Unterseite voran auf die Tibiavorderkante aufgestellt. Bei einer Ausgestaltung weist die Unterseite die wenigstens zwei Stützgeometrien auf. Diese können beispielsweise jeweils gemäß der vorhergehenden Ausgestaltung gabelförmig posterior von der Unterseite abragen. Bei einer weiteren Ausgestaltung bildet die Unterseite selbst die wenigstens zwei Stützgeometrien. In diesem Fall definieren zwei proximodistal voneinander beabstandete "Punkte", Linien oder Flächenabschnitte der Unterseite die Stützgeometrien. Die Platte kann eine beliebige Grundform aufweisen, wobei eine runde, rechteckige oder dreieckige Grundform bevorzugt sind.

In weiterer Ausgestaltung der Erfindung weist die Platte eine an der Oberseite angeordnete Skale mit distal aufgefächerten Teilstrichen auf, wobei die Teilstriche jeweils einen Wert eines Varus/Valgus-Winkels der Tibia repräsentieren. Die Skale ermöglicht ein einfaches Ablesen und/oder Einstellen des Varus/Valgus-Winkels. Durch die Anbringung an der Oberseite kann der ausführende Operateur die Skale besonders einfach und zuverlässig ablesen.

Die Erfindung betrifft zudem eine Anordnung mit einer chirurgischen Vorrichtung gemäß der vorhergehenden Beschreibung und mit einem extramedullären Ausrichtstab. Hinsichtlich der Merkmale und Vorteile der chirurgischen Vorrichtung wird auf die vorhergehende Beschreibung verwiesen. Der extramedulläre Ausrichtstab ist zwischen einem ersten Ende und einem zweiten Ende längserstreckt. Vorzugsweise ist eines der beiden Enden zur Befestigung an einem tibialen Ausricht- und/oder Übertragungswerkzeug eingerichtet.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivdarstellung eine Ausführungsform einer erfindungsgemäßen chirurgischen Vorrichtung,
- Fig. 2: in schematischer Perspektivdarstellung eine exemplarische intraoperative Situation, in welcher die chirurgische Vorrichtung zum Ausrichten eines extramedullären Ausrichtstabs an einer Tibiavorderkante verwendet wird,
- Fig. 3: die chirurgische Vorrichtung nach den Fig. 1 und 2 in einer schematischen Seitenansicht mit medialer Blickrichtung,
- Fig. 4: die chirurgische Vorrichtung nach den Fig. 1 bis 3 in einer schematischen Schnittdarstellung entlang einer Schnittlinie A-A nach Fig. 3,
- Fig. 5: die chirurgische Vorrichtung nach den Fig. 1 bis 4 in einer schematischen Rückansicht mit distaler Blickrichtung,
- Fig. 6: eine vergrößerte Detaildarstellung eines Bereichs B nach Fig. 5,
- Fig. 7: in schematischer Perspektivansicht eine weitere Ausführungsform einer erfindungsgemäßen chirurgischen Vorrichtung,
- Fig. 8: in schematischer Perspektivdarstellung eine exemplarische intraoperative Situation, in welcher die chirurgische Vorrichtung nach Fig. 7 zum Ausrichten eines extramedullären Ausrichtstabs an einer Tibiavorderkante verwendet wird,
- Fig. 9: eine teilweise abgeschnittene, perspektivische Detaildarstellung zur Verdeutlichung weiterer Merkmale,
- Fig. 10: in schematischer Seitenansicht eine Ausführungsform eines extramedullären Ausrichtstabs,
- Fig. 11: in schematischer Perspektivdarstellung einen Detailbereich des extramedullären Ausrichtstabs nach Fig. 10 und
- Fig. 12, 13: Detaildarstellungen zur Verdeutlichung weiterer Merkmale des extramedullären Ausrichtstabs und/oder der chirurgischen Vorrichtungen.

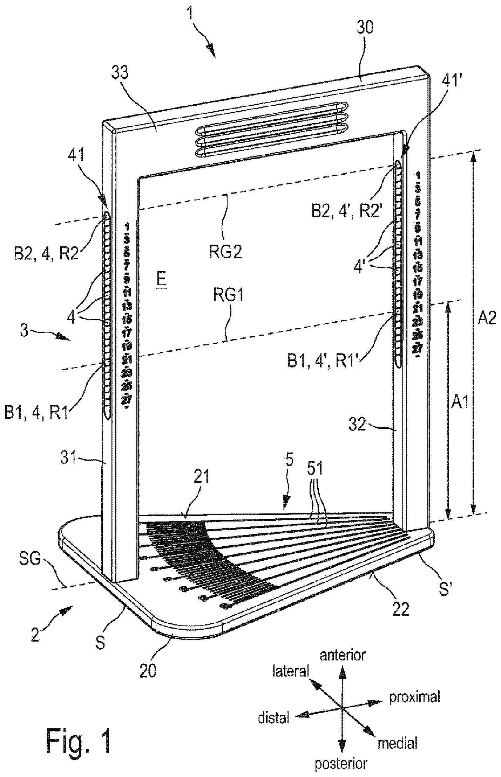

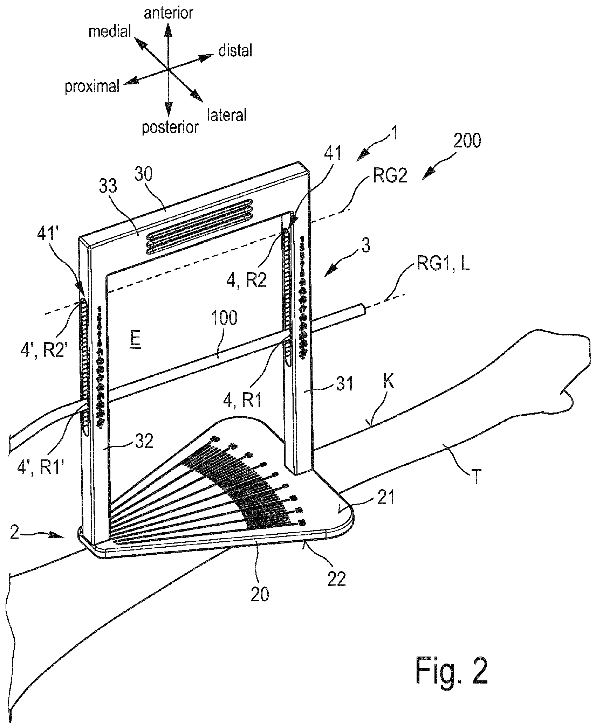

Gemäß den Fig. 1 bis 6 ist eine chirurgische Vorrichtung 1 zur Verwendung bei einer Kniegelenkersatzoperation vorgesehen. Die chirurgische Vorrichtung 1 dient zum Ausrichten eines extramedullären Ausrichtstabs 100. Die chirurgische Vorrichtung 1 und der extramedulläre Ausrichtstab 100 bilden eine Anordnung 200 (Fig. 2).

Die chirurgische Vorrichtung 1 weist einen Stützabschnitt 2 und einen Referenzabschnitt 3 auf.

Der Stützabschnitt 2 ist bei der Ausführungsform nach den Fig. 1 bis 6 als Platte 20 ausgebildet.

Die Platte 20 weist eine anterior orientierte Oberseite 21 und eine posterior orientierte Unterseite 22 auf. Die Unterseite 22 ist eben und zum Auflegen auf eine anteriore Kante K einer Tibia T eingerichtet (siehe Fig. 2). Die Unterseite 22 ist mediolateral und proximodistal erstreckt. Dabei bilden einander proximodistal gegenüberliegende Enden und/oder Punkte der Unterseite 22 zwei (gedachte) Stützgeometrien S, S'. Diese beiden Stützgeometrien S, S' sind entlang einer (gedachten) Stützgeraden SG voneinander beabstandet. Die Stützgerade SG ist proximodistal erstreckt. Die Stützgeometrie S kann auch als distale Stützgeometrie bezeichnet werden. Die Stützgeometrie S' kann auch als proximale Stützgeometrie S' bezeichnet werden.

Der Referenzabschnitt 3 ragt anterior von dem Stützabschnitt 2 auf. Im Hinblick auf die vorliegende Ausführungsform ragt der Referenzabschnitt 3 anterior von der Oberseite 21 der Platte 20 auf. Der Referenzabschnitt 3 weist wenigstens zwei Referenzgeometrien R1, R1' auf. Die besagten Referenzgeometrien R1, R1' sind entlang einer (gedachten) proximodistal erstreckten Referenzgeraden RG1 voneinander beabstandet. Die Referenzgeometrie R1 kann auch als distale Referenzgeometrie R1 bezeichnet werden. Die Referenzgeometrie R1' kann auch als proximale Referenzgeometrie bezeichnet werden.

Die Referenzgerade RG1 ist um einen anterioren Abstand A1 von der Stützgeraden SG beabstandet und parallel zu derselben erstreckt. In der Verwendung der chirurgischen Vorrichtung 1 dient die Referenzgerade RG1 als Referenz zur Ausrichtung des extramedullären Ausrichtstabs 100 (siehe Fig. 2). Der extramedulläre Ausrichtstab 100 kann über eine parallele und/oder koaxiale Ausrichtung entlang der Referenzgeraden RG1 mittelbar parallel zu der anterioren Kante K der Tibia T ausgerichtet werden. Die beiden Referenzgeometrien R1, R1' sind jeweils zur formschlüssigen Lagerung des extramedullären Ausrichtstabs 100 eingerichtet. Die formschlüssige Lagerung wirkt einer ungewollten Relativbewegung zwischen dem extramedullären Ausrichtstab 100 und der chirurgischen Vorrichtung 1 entgegen.

Bei der gezeigten Ausführungsform weist der Referenzabschnitt 3 zwei weitere Referenzgeometrien R2, R2' auf. In diesem Zusammenhang kann auch von zwei ersten Referenzgeometrien R1, R1' und zwei zweiten Referenzgeometrien R2, R2' gesprochen werden. Die beiden zweiten Referenzgeometrien R2, R2' sind entlang einer weiteren Referenzgeraden RG2 proximodistal voneinander beabstandet. Die Referenzgerade RG1 und die weitere Referenzgerade RG2 werden nachfolgend auch als erste Referenzgerade RG1 und zweite Referenzgerade RG2 bezeichnet. Die zweite Referenzgerade RG2 ist parallel zu der ersten Referenzgeraden RG1 - und damit auch parallel zu der Stützgeraden SG - ausgerichtet. Die zweite Referenzgerade RG2 ist in einem anterioren zweiten Abstand A2 von der Stützgeraden SG beabstandet. Der zweite Abstand A2 ist vorliegend größer als der (erste) Abstand A1 der ersten Referenzgeraden RG1. Die erste Referenzgerade RG1 und die zweite Referenzgerade RG2 sind bei der gezeigten Ausführungsform in einer gemeinsamen sagittalen Ebene E angeordnet. Auch die Stützgerade SG liegt in der sagittalen Ebene E.

Bei der gezeigten Ausführungsform sind die Referenzgeometrien R1, R1', R2, R2' jeweils als Bohrung 4, 4' ausgebildet. Die Bohrungen 4, 4' können auch als distale Bohrungen 4 und proximale Bohrungen 4' bezeichnet werden. Hinsichtlich der jeweiligen Zuordnung zu der ersten Referenzgeraden RG1 oder der zweiten Referenzgeraden RG2 kann auch von ersten Bohrungen B1 und zweiten Bohrungen B2 gesprochen werden. Sämtliche Bohrungen sind vorliegend identisch ausgeführt und/oder proximodistal längserstreckt. Die ersten Bohrungen B1 sind jeweils koaxial zu der ersten Referenzgeraden RG1 längserstreckt. Die zweiten Bohrungen B2 sind jeweils koaxial zu der zweiten Referenzgeraden RG2 längserstreckt.

Bei der gezeigten Ausführungsform sind nicht lediglich zwei erste und zwei zweite Bohrungen, sondern stattdessen eine Vielzahl proximodistal paarweise angeordneter Bohrungen vorhanden. Die Vielzahl an Bohrungen bilden eine distale Bohrungsreihe 41 und eine proximale Bohrungsreihe 41'. Bei der gezeigten Ausführungsform sind jeweils 27 proximale und distale Bohrungen und somit 27 Bohrungspaare vorhanden. Jedem Bohrungspaar ist eine Referenzgerade und dementsprechend auch ein anteriorer Abstand zu der Stützgeraden SG zugeordnet.

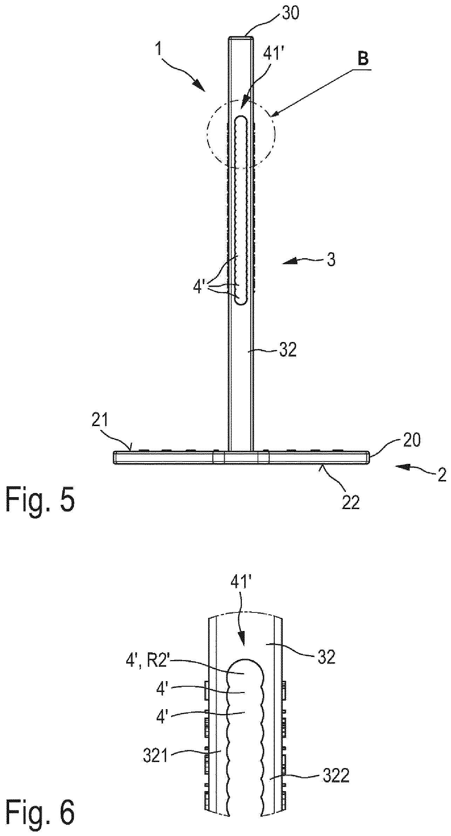

Die proximalen Bohrungen 4' sind anteroposterior aneinandergereiht, wobei unmittelbar benachbarte Bohrungen jeweils miteinander verbunden sind (siehe auch Fig. 6). Entsprechendes gilt sinngemäß für die distalen Bohrungen 4 der distalen Bohrungsreihe 41. Der extramedulläre Ausrichtstab 100 ist an seinem Außenumfang formschlüssig in den beiden Bohrungsreihen 41, 41' gehalten. Durch die besagte Verbindung zwischen den Bohrungen 4, 4' kann der extramedulläre Ausrichtstab 100 in anteroposteriore Richtung zwischen den einzelnen Bohrungspaaren der Bohrungsreihen 41, 41' verschoben werden.

In der anhand Fig. 2 gezeigten Situation ist der extramedulläre Ausrichtstab 100 in einer ersten Position formschlüssig gelagert. In dieser ersten Position ist der extramedulläre Ausrichtstab 100 koaxial zu der ersten Referenzgeraden RG1 ausgerichtet und an den der besagten Referenzgeraden RG1 zugeordneten ersten Bohrungen B1 der Bohrungsreihen 41, 41' formschlüssig gehalten. Der Formschluss wirkt hierbei mediolateral. In proximodistaler Richtung ist der extramedulläre Ausrichtstab 100 gleitbeweglich in den Bohrungsreihen 41, 41' gehalten. Ausgehend von der gezeigten ersten Position kann der extramedulläre Ausrichtstab 100 in Längsrichtung der Bohrungsreihen 41, 41' anterior in Richtung der zweiten Referenzgeraden RG2 verschoben werden. Hierbei überrastet der Außenumfang des extramedullären Ausrichtstabs 100 die einzelnen Bohrungen 4, 4' der beiden Bohrungsreihen 41, 41'.

Bei der gezeigten Ausführungsform ist der Referenzabschnitt 2 im Bereich der Bohrungen 4, 4' und/oder Bohrungsreihen 41, 41' elastisch formnachgiebig. Die formnachgiebigen Eigenschaften werden vorliegend auf noch näher beschriebene Weise erreicht. Die elastische Formnachgiebigkeit ermöglicht es, dass der extramedulläre Ausrichtstab 100 auf die vorbeschriebene Weise von einem Bohrungspaar in ein benachbartes Bohrungspaar "gerastet" werden kann.

Bei der gezeigten Ausführungsform ist der Referenzabschnitt als Bügel 30 ausgebildet.

Der Bügel 30 weist vorliegend zwei Längsschenkel 31, 32 und einen Querschenkel 33 auf.

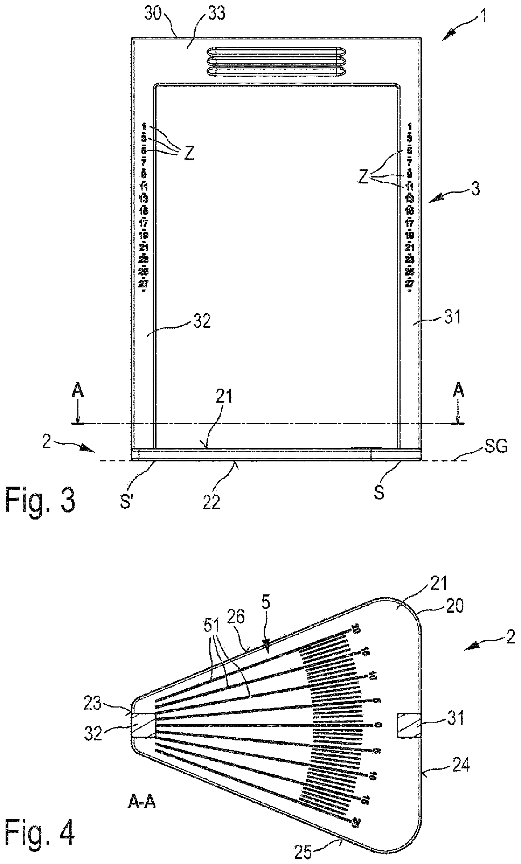

Die Längsschenkel 31, 32 können auch als distaler Längsschenkel 31 und proximaler Längsschenkel 32 bezeichnet werden. Die beiden Längsschenkel 31, 32 sind jeweils anteroposterior längserstreckt. Vorliegend sind die beiden Längsschenkel 31, 32 parallel zueinander ausgerichtet. Die Längsschenkel 31, 32 sind proximodistal voneinander beabstandet. Die Längsschenkel 31, 32 ragen anterior von dem Stützabschnitt 2 auf. Im Speziellen ragen die Längsschenkel 31, 32 vorliegend von der Oberseite 21 der Platte 20 auf. Vorliegend sind die Längsschenkel 31, 32 orthogonal zu der Oberseite 21 ausgerichtet. Die Längsschenkel 31, 32 weisen vorliegend jeweils eine rechteckige, im Speziellen quadratische, Querschnittsform auf (siehe Fig. 4). Zur verbesserten Anzeige und leichten Erkennbarkeit der jeweiligen Position des extramedullären Ausrichtstabs 100 sind die Längsschenkel 31, 32 vorliegend jeweils mit Markierungsziffern Z versehen. Die Markierungsziffern Z sind bei der gezeigten Ausführungsform von 1 bis 27 durchnummeriert.

Der Querschenkel 33 ist proximodistal zwischen den beiden Längsschenkeln 31, 32 längserstreckt. Der Querschenkel 33 ist einends der beiden Längsschenkel 31, 32 angeordnet. Der Stützabschnitt 2, im Speziellen die Platte 20, ist vorliegend andernends der Längsschenkel 31, 32 angeordnet. Der Querschenkel 33 ist parallel zu der Oberseite 21 und/oder orthogonal zu den beiden Längsschenkeln 31, 32 orientiert.

Vorliegend sind die proximalen Bohrungen 4' und/oder ist die proximale Bohrungsreihe 41' in den proximalen Längsschenkel 32 eingebracht. Die distalen Bohrungen 4 und/oder die distale Bohrungsreihe 41 sind in den distalen Längsschenkel 31 eingebracht.

Die Bohrungen 4, 4' sind durch den Querschnitt des jeweiligen Längsschenkels 31, 32 erstreckt. Die Längsschenkel 31, 32 sind im Bereich der Bohrungsreihen 41, 41' in mediolateraler Richtung dünnwandig (siehe Fig. 5, 6). Die Bohrungen 4' der Bohrungsreihe 41 sind vorliegend in mediolateraler Richtung von einer medialen Wange 321 und einer lateralen Wange 322 des proximalen Längsschenkels 32 begrenzt (siehe Fig. 6). Die besagten Wangen 321, 322 sind dünnwandig und folglich im Vergleich zu den übrigen Bereichen des Längsschenkels 32 elastisch formnachgiebig. Die elastische Formnachgiebigkeit ermöglicht ein anforderungsgerechtes Verrasten und Bewegen des extramedullären Ausrichtstabs 100. Das diesbezüglich zu dem proximalen Längsschenkel 32 Offenbarte gilt mutatis mutandis auch für den distalen Längsschenkel 31.

Bei der Ausführungsform nach den Fig. 1 bis 6 weist die Oberseite 21 der Platte 20 eine Skale 5 auf. Die Skale 5 weist Teilstriche 51 auf. Die Teilstriche 51 sind in distaler Richtung aufgefächert und repräsentieren jeweils einen Wert eines Varus/Valgus-Winkels der Tibia T. An der Skale 5 kann der Varus/Valgus-Winkel der Tibia T abgelesen werden. Vorliegend entspricht eine Ausrichtung der anterioren Kante K in der sagittalen Ebene E einem Varus/Valgus-Winkel von 0° (siehe Fig. 4).

Die Platte 20 ist vorliegend in ihrer Formgebung an ihre Funktion als Anzeigefläche für die Skale 5 angepasst. Im Speziellen weist die Platte 20 eine proximale Kante 23, eine gegenüberliegende distale Kante 24, eine laterale Kante 25 und eine gegenüberliegende mediale Kante 26 auf. Die proximale Kante 23 und die distale Kante 24 sind zueinander parallel. Die proximale Kante 23 ist kürzer als die distale Kante 24. Die laterale Kante 25 ist ausgehend von der proximalen Kante 23 in distaler Richtung lateral nach außen geneigt. Die mediale Kante ist hierzu symmetrisch medial nach außen geneigt. Die laterale Kante 25 und die mediale Kante 26 sind dementsprechend nicht parallel zueinander.

Bei einer in den Figuren nicht gezeigten Ausführungsform ist der Referenzabschnitt nicht etwa als Bügel, sondern stattdessen als eine Art Platte gestaltet. Die Referenzgeometrien sind bei dieser Ausführungsform nicht etwa als Bohrung, sondern stattdessen als medial oder lateral offene Rastaufnahme gestaltet. Die vorliegende Gestaltung des Referenzabschnitts 3 als Bügel 30 ist insoweit als vorteilhaft, aber nicht als wesentlich im Hinblick auf die vorliegende Erfindung zu erachten.

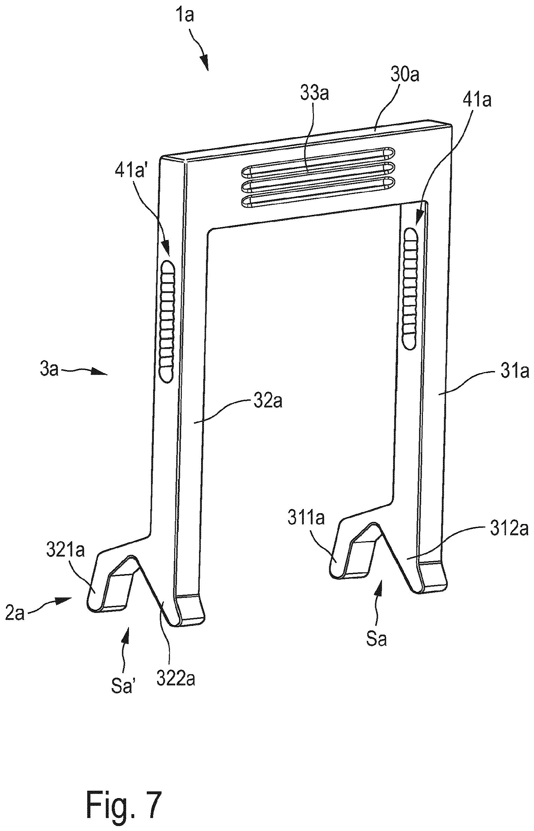

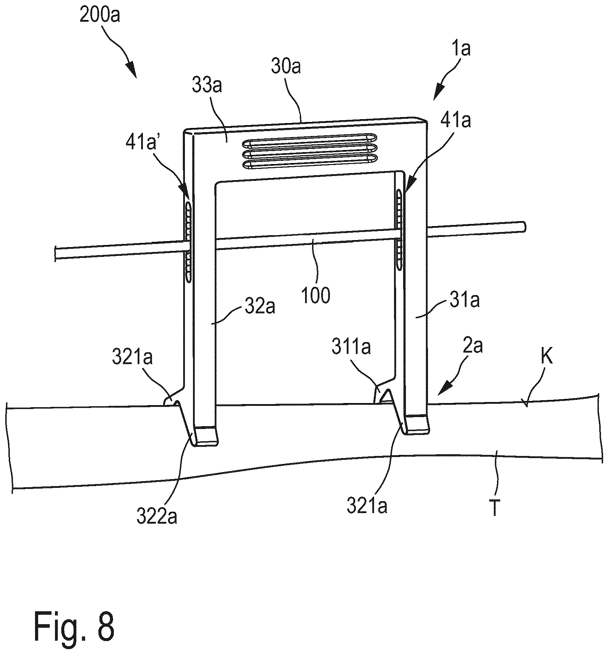

Die Fig. 7 und 8 zeigen eine weitere Ausführungsform einer erfindungsgemäßen chirurgischen Vorrichtung 1a. Die chirurgische Vorrichtung 1a ist im Wesentlichen identisch zu der chirurgischen Vorrichtung 1 nach den Fig. 1 bis 6. Zur Vermeidung von Wiederholungen werden nachfolgend lediglich wesentliche Unterschiede erörtert. Bauteile und/oder Abschnitte der chirurgischen Vorrichtung 1a nach den Fig. 7 und 8, die mit identischer Gestaltung und/oder Funktion auch bei der chirurgischen Vorrichtung 1 nach den Fig. 1 bis 6 vorhanden sind, werden nicht gesondert erläutert. Stattdessen wird auf die Offenbarung zu der chirurgischen Vorrichtung 1 verwiesen und ausdrücklich Bezug genommen.

Die chirurgische Vorrichtung 1a weist einen unterschiedlich gestalteten Stützabschnitt 2a auf. Dieser ist nicht etwa als Platte, sondern stattdessen durch die Stirnenden der Längsschenkel 31a, 32a gebildet. Dabei bildet ein posteriores Stirnende des distalen Längsschenkels 31a vorliegend die distale Stützgeometrie Sa. Das entsprechende Stirnende des proximalen Längsschenkels 32a bildet die proximale Stützgeometrie Sa'.

Bei der gezeigten Ausführungsform weisen die Stirnenden und/oder Stützgeometrien Sa, Sa' jeweils eine Gabelform mit einem ersten Astabschnitt 311a, 321a und einem zweiten Astabschnitt 312a, 322a auf. Mit anderen Worten ausgedrückt, sind die Längsschenkel 31a, 32a einends jeweils in die besagten Astabschnitte 311a, 312a bzw. 321a, 322a verzweigt. Die verzweigte und/oder gabelförmige Gestaltung wirkt einem Abrutschen der chirurgischen Vorrichtung 1 von der anterioren Kante K entgegen (siehe Fig. 8). Die chirurgische Vorrichtung 1a bildet zusammen mit dem extramedullären Ausrichtstab 100 eine weitere Anordnung 200a.

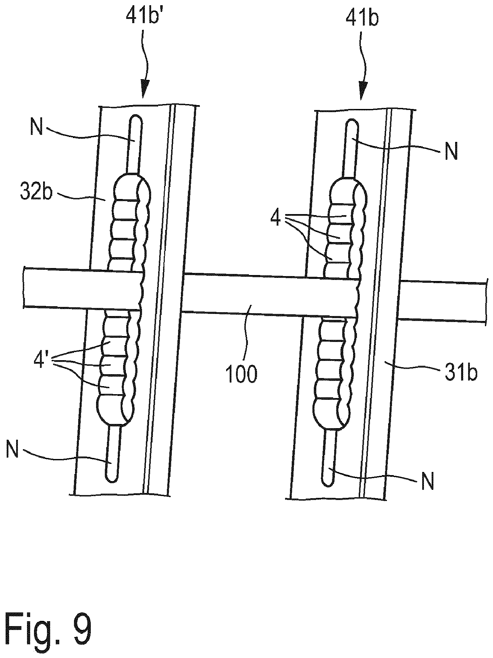

Anhand der Fig. 9 bis 13 sind weitere gegenständliche und/oder funktionelle Merkmale gezeigt. Die dort gezeigten Merkmale sind jeweils einzeln oder in Kombination mit den Merkmalen der chirurgischen Vorrichtungen 1, 1a und/oder Anordnungen 200, 200a kombinierbar.

Fig. 9 zeigt Bohrungsreihen 41b, 41b'. Die Bohrungsreihen 41b, 41b' sind im Wesentlichen identisch mit den Bohrungsreihen 41a, 41a' der chirurgischen Vorrichtung 1a und den Bohrungsreihen 41, 41' der chirurgischen Vorrichtung 1. Die Bohrungsreihen 41b, 41b' weisen an ihren gegenüberliegenden Stirnenden jeweils einen Längsschlitz N auf. Die Längsschlitze N unterstützen die bereits erläuterte elastische Formnachgiebigkeit der Längsschenkel 31b, 32b. Die Längsschlitze N sind insbesondere dann vorteilhaft, wenn die chirurgische Vorrichtung im Bereich der Bohrungsreihen dickwandig und/oder aus Metall gefertigt ist.

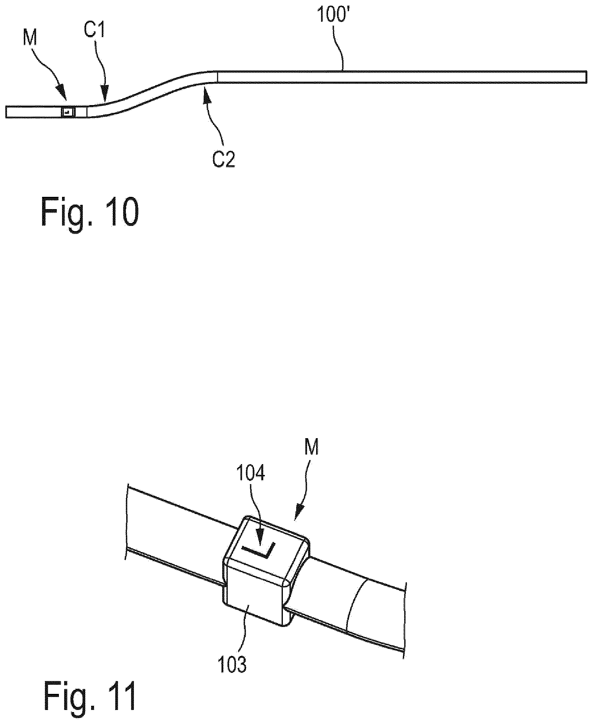

Die Fig. 10 und 11 zeigen einen unterschiedlich gestalteten extramedullären Ausrichtstab 100'. Der extramedulläre Ausrichtstab 100' kann anstelle des extramedullären Ausrichtstabs 100 jeweils gemeinsam mit einer der chirurgischen Vorrichtungen 1, 1a verwendet werden. Der Ausrichtstab 100' weist eine erste Biegung C1 und eine zweite Biegung C2 auf (siehe Fig. 10). Die beiden Biegungen C1, C2 untergliedern den Ausrichtstab 100' in zwei nicht näher bezeichnete parallel versetzt längserstreckte Abschnitte. Der Ausrichtstab 100' weist zudem eine Markierung M in Form eines quaderförmigen Elements 103 auf. Das quaderförmige Element 103 ist mit einem Zeichen 104 versehen. Das Zeichen 104 ist vorliegend der Buchstabe "L".

Bei der gezeigten Ausführungsform ist das quaderförmige Element 103 auf seiner dem Zeichen 104 gegenüberliegenden Seite mit einem in den Figuren nicht ersichtlichen weiteren Zeichen (ohne Bezugszeichen) versehen. Das weitere Zeichen ist vorliegend der Buchstabe "R".

Der extramedulläre Ausrichtstab 100' kann derart eingesteckt werden, dass das Zeichen 104 ("L") anterior ausgerichtet ist. In diesem Fall ist der extramedulläre Ausrichtstab 100' zur Verwendung am linken Bein eingerichtet. Der extramedulläre Ausrichtstab 100' kann zudem um 180° um seine Längsachse gedreht eingesteckt werden, so dass das weitere Zeichen ("R") anterior - und das Zeichen 104 posterior - ausgerichtet ist. In diesem Fall ist der extramedulläre Ausrichtstab 100' zur Verwendung am rechten Bein eingerichtet.

Die Markierung M zeigt dem Chirurgen mithin an, zur Verwendung an welchem Bein der extramedulläre Ausrichtstab 100' ausgerichtet ist.

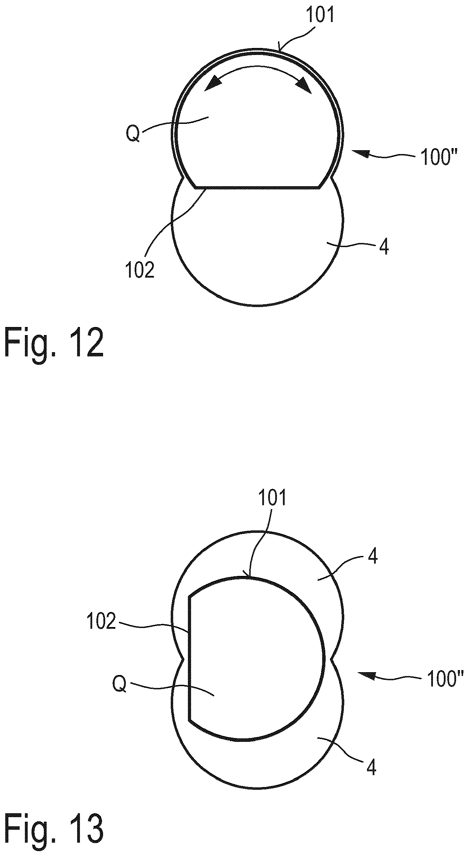

Die Fig. 12 und 13 zeigen Merkmale eines weiteren Ausrichtstabs 100". Der Ausrichtstab 100" ist in proximaler Blickrichtung auf seinen Querschnitt Q gezeigt. Der Querschnitt Q ist nichtrotationssymmetrisch. Hierzu im Unterschied sind die Querschnitte (ohne Bezugszeichen) der extramedullären Ausrichtstäbe 100, 100' rotationssymmetrisch, im Speziellen kreisrund. Der Außenumfang 101 des Ausrichtstabs 100" weist eine radiale Abflachung 102 auf. In der anhand Fig. 12 gezeigten Situation ist der Ausrichtstab 100" in mediolateraler und anteroposterior Richtung formschlüssig in einer oberen der beiden Bohrungen 4 gehalten. Bei der gezeigten Ausführungsform sind die Bohrungen 4 in radialer Richtung nicht oder jedenfalls nicht ausreichend elastisch formnachgiebig, um eine rastbewegliche Verlagerung des Ausrichtstabs 100" zu ermöglichen. Zur Bewegung des Ausrichtstabs 100" von der oberen in die untere der beiden Bohrungen 4 wird der Ausrichtstab 100' um seine Längsachse gedreht. Ausgehend von der in Fig. 12 gezeigten Position erfolgt eine Drehung um 90° im Uhrzeigersinn. Alternativ kann eine Drehung um 90° entgegen dem Uhrzeigersinn erfolgen. Durch die veränderte Position der radialen Abflachung 102 wird ein wirksamer Durchmesser des Ausrichtstabs 100" verändert. Der Ausrichtstab 100" kann in der insoweit gedrehten Position ohne weiteres von der oberen in die untere der beiden Bohrungen 4 verlagert werden. Zur formschlüssigen Lagerung an der unteren der beiden Bohrungen 4 wird der Ausrichtstab 100" wiederum um seine Längsachse gedreht, so dass die radiale Abflachung 102 entweder in anteriore Richtung oder posteriore Richtung weist.

## Patentansprüche

1. Chirurgische Vorrichtung (1, 1a) zum Ausrichten eines extramedullären Ausrichtstabs (100, 100', 100") bei einer Kniegelenkersatzoperation, aufweisend
einen Stützabschnitt (2, 2a) mit wenigstens zwei Stützgeometrien (S, S', Sa, Sa'), welche entlang einer proximodistal erstreckten Stützgeraden (SG) voneinander beabstandet und jeweils zum Abstützen auf einer anterioren Kante (K) einer Tibia (T) eingerichtet sind, und
einen von dem Stützabschnitt (2, 2a) anterior aufragenden Referenzabschnitt (3, 3a) mit wenigstens zwei Referenzgeometrien (R1, R1', R2, R2'), welche entlang einer proximodistal erstreckten Referenzgeraden (RG1, RG2) voneinander beabstandet sind,
wobei die Referenzgerade (RG1, RG2) um einen anterioren Abstand (A1, A2) von der Stützgeraden (SG) beabstandet und parallel zu der Stützgeraden (SG) erstreckt ist,
wobei die wenigstens zwei Referenzgeometrien (R1, R1', R2, R2') und/oder die Referenzgerade (RG1, RG2) anstelle der anterioren Kante (K) der Tibia (T) als Referenz zum Ausrichten des extramedullären Ausrichtstabs (100, 100', 100") dienen,
und wobei die wenigstens zwei Referenzgeometrien (R1, R1', R2, R2') jeweils zur wenigstens mediolateral formschlüssigen Lagerung des extramedullären Ausrichtstabs (100, 100', 100") eingerichtet sind,
**dadurch gekennzeichnet, dass** der Referenzabschnitt (3, 3a) wenigstens zwei erste Referenzgeometrien (R1, R1'), welche entlang einer ersten Referenzgeraden (RG1) voneinander beabstandet sind, und zwei zweite Referenzgeometrien (R2, R2') aufweist, welche entlang einer zweiten Referenzgeraden (RG2) voneinander beabstandet sind, wobei die erste Referenzgerade (RG1) in einem anterioren ersten Abstand (A1) von der Stützgeraden (SG) beabstandet ist, und wobei die zweite Referenzgerade (RG2) in einem anterioren zweiten Abstand (A2) von der Stützgeraden (SG) beabstandet ist.

2. Chirurgische Vorrichtung (1, 1a) nach Anspruch 1, **dadurch gekennzeichnet, dass** die Referenzgeometrien (R1, R1', R2, R2') jeweils als Bohrung (4, 4') ausgebildet sind, so dass wenigstens zwei erste Bohrungen (B1) und zwei zweite Bohrungen (B2) vorhanden sind.

3. Chirurgische Vorrichtung (1, 1a) nach Anspruch 2, **dadurch gekennzeichnet, dass** die ersten Bohrungen (B1) und die zweiten Bohrungen (B2) anteroposterior miteinander verbunden sind, wodurch der extramedulläre Ausrichtstab (100, 100', 100") ausgehend von einer Lagerung an den zwei ersten Bohrungen (B1) anteroposterior und/oder in seiner Radialrichtung verschiebbar ist in eine Lagerung an den zwei zweiten Bohrungen (B2) und umgekehrt.

4. Chirurgische Vorrichtung (1, 1a) nach Anspruch 2 oder 3, **dadurch gekennzeichnet, dass** der Referenzabschnitt (3, 3a) im Bereich der Bohrungen (4, 4') elastisch formnachgiebig ist.

5. Chirurgische Vorrichtung (1, 1a) nach einem der vorhergehenden Ansprüche, **dadurch gekennzeichnet, dass** der Referenzabschnitt (3, 3a) als Bügel (30, 30a) mit zwei Längsschenkeln (31, 32, 31a, 32a) und einem Querschenkel (33, 33a) ausgebildet ist, wobei die zwei Längsschenkel (31, 32, 31a, 32a) proximodistal voneinander beabstandet sowie jeweils anteroposterior längserstreckt sind und die Referenzgeometrien (R1, R1', R2, R2') aufweisen, und wobei der Querschenkel (33, 33a) proximodistal zwischen den zwei Längsschenkeln (31, 32, 31a, 32a) längserstreckt ist und dieselben miteinander verbindet.

6. Chirurgische Vorrichtung (1, 1a) nach Anspruch 5, **dadurch gekennzeichnet, dass** die Längsschenkel (31, 32, 31a, 32a) jeweils eine Bohrungsreihe (41, 41', 41a, 41a') mit einer Vielzahl von anteroposterior aneinandergereihten Bohrungen (4, 4') als Referenzgeometrien aufweisen.

7. Chirurgische Vorrichtung (1a) nach Anspruch 5 oder 6, **dadurch gekennzeichnet, dass** der Stützabschnitt (2a) durch Stirnenden der Längsschenkel (31a, 32a) gebildet ist, wobei jeweils ein Stirnende eine der Stützgeometrien (Sa, Sa') aufweist.

8. Chirurgische Vorrichtung (1a) nach Anspruch 7, **dadurch gekennzeichnet, dass** die Stützgeometrien (Sa, Sa') jeweils eine Gabelform mit einem ersten Astabschnitt (311a, 321a) und einem zweiten Astabschnitt (312a, 322a) aufweisen.

9. Chirurgische Vorrichtung (1) nach einem der Ansprüche 1 bis 6, **dadurch gekennzeichnet, dass** der Stützabschnitt (2) als Platte (20) mit einer posterior orientierten Unterseite (22) und einer anterior orientierten Oberseite (21) ausgebildet ist, wobei die Unterseite (22) eben ist und die wenigstens zwei Stützgeometrien (S, S') aufweist und/oder bildet, und wobei der Referenzabschnitt (3) von der Oberseite (21) aufragt.

10. Chirurgische Vorrichtung (1) nach Anspruch 9, **dadurch gekennzeichnet, dass** die Platte (20) eine an der Oberseite (21) angeordnete Skale (5) mit distal aufgefächerten Teilstrichen (51) aufweist, wobei die Teilstriche (51) jeweils einen Wert eines Varus/Valgus-Winkels der Tibia (T) repräsentieren.

11. Anordnung (200, 200a) mit einer chirurgischen Vorrichtung (1, 1a) nach einem der vorhergehenden Ansprüche und mit einem extramedullären Ausrichtstab (100, 100', 100").

## Claims

1. Surgical device (1, 1a) for aligning an extramedullary alignment rod (100, 100', 100'') in a knee joint replacement operation, having
a supporting section (2, 2a) with at least two supporting geometries (S, S', Sa, Sa'), which are spaced apart from one another along a supporting line (SG) that extends in a proximodistal direction and are each configured for supporting on an anterior edge (K) of a tibia (T), and
a reference section (3, 3a), which projects in an anterior direction from the supporting section (2, 2a) and has at least two reference geometries (R1, R1', R2, R2'), which are spaced apart from one another along a reference line (RG1, RG2) that extends in a proximodistal direction,
wherein the reference line (RG1, RG2) is spaced apart from the supporting line (SG) by an anterior distance (A1, A2) and extends parallel to the supporting line (SG),
wherein the at least two reference geometries (R1, R1', R2, R2') and/or the reference line (RG1, RG2) are used, instead of the anterior edge (K) of the tibia (T), as a reference for aligning the extramedullary alignment rod (100, 100', 100''),
and wherein the at least two reference geometries (R1, R1', R2, R2') are each configured for the at least mediolaterally form-fit mounting of the extramedullary alignment rod (100, 100', 100''),
**characterized in that** the reference section (3, 3a) has at least two first reference geometries (R1, R1'), which are spaced apart from one another along a first reference line (RG1), and two second reference geometries (R2, R2'), which are spaced apart from one another along a second reference line (RG2), wherein the first reference line (RG1) is spaced apart from the supporting line (SG) by an anterior first distance (A1), and wherein the second reference line (RG2) is spaced apart from the supporting line (SG) by an anterior second distance (A2).

2. Surgical device (1, 1a) according to Claim 1, **characterized in that** the reference geometries (R1, R1', R2, R2') are each designed as a bore (4, 4'), such that there are at least two first bores (B1) and two second bores (B2).

3. Surgical device (1, 1a) according to Claim 2, **characterized in that** the first bores (B1) and the second bores (B2) are connected to one another in an anteroposterior direction, as a result of which the extramedullary alignment rod (100, 100', 100''), starting from being mounted on the two first bores (B1), can be moved in an anteroposterior direction and/or in its radial direction to being mounted on the two second bores (B2), and vice versa.

4. Surgical device (1, 1a) according to Claim 2 or 3, **characterized in that** the reference section (3, 3a) is resiliently flexible in the region of the bores (4, 4').

5. Surgical device (1, 1a) according to any one of the preceding claims, **characterized in that** the reference section (3, 3a) is designed as a frame (30, 30a) having two longitudinal branches (31, 32, 31a, 32a) and one transverse branch (33, 33a), wherein the two longitudinal branches (31, 32, 31a, 32a) are spaced apart from one another in a proximodistal direction and each extend lengthwise in an anteroposterior direction and have the reference geometries (R1, R1', R2, R2'), and wherein the transverse branch (33, 33a) extends lengthwise in a proximodistal direction between the two longitudinal branches (31, 32, 31a, 32a) and connects them to one another.

6. Surgical device (1, 1a) according to Claim 5, **characterized in that** the longitudinal branches (31, 32, 31a, 32a) each have a row of bores (41, 41', 41a, 41a'), each row having a multiplicity of bores (4, 4') following one another in an anteroposterior direction as reference geometries.

7. Surgical device (1a) according to Claim 5 or 6, **characterized in that** the supporting section (2a) is formed by extreme ends of the longitudinal branches (31a, 32a), wherein each extreme end respectively has one of the supporting geometries (Sa, Sa').

8. Surgical device (1a) according to Claim 7, **characterized in that** the supporting geometries (Sa, Sa') each have a fork shape with a first prong section (311a, 321a) and a second prong section (312a, 322a).

9. Surgical device (1) according to any one of Claims 1 to 6, **characterized in that** the supporting section (2) is designed as a plate (20) having a lower side (22) oriented in a posterior direction and an upper side (21) oriented in an anterior direction, wherein the lower side (22) is plane and has and/or forms the at least two supporting geometries (S, S'), and wherein the reference section (3) projects from the upper side (21).

10. Surgical device (1) according to Claim 9, **characterized in that** the plate (20) has a scale (5) arranged on the upper side (21), with graduations (51) fanning out in a distal direction, wherein the graduations (51) each represent a value of a varus/valgus angle of the tibia (T).

11. Arrangement (200, 200a) having a surgical device (1, 1a) according to any one of the preceding claims and having an extramedullary alignment rod (100, 100', 100'').

## Revendications

1. Dispositif chirurgical (1, 1a) pour aligner une tige d'alignement extramédullaire (100, 100', 100") lors d'une opération de remplacement de l'articulation du genou, présentant
une section d'appui (2, 2a) avec au moins deux géométries d'appui (S, S', Sa, Sa') qui sont espacées l'une de l'autre le long d'une droite d'appui (SG) s'étendant proximodistalement et qui sont chacune adaptées pour s'appuyer sur un bord antérieur (K) d'un tibia (T), et
une section de référence (3, 3a) faisant saillie antérieurement depuis la section d'appui (2, 2a), avec au moins deux géométries de référence (R1, R1', R2, R2') qui sont espacées l'une de l'autre le long d'une droite de référence (RG1, RG2) s'étendant proximodistalement,
la droite de référence (RG1, RG2) étant espacée de la droite d'appui (SG) d'une distance antérieure (A1, A2) et s'étendant parallèlement à la droite d'appui (SG),
les au moins deux géométries de référence (R1, R1', R2, R2') et/ou la droite de référence (RG1, RG2) servant de référence pour orienter la tige d'alignement extramédullaire (100, 100', 100'') à la place du bord antérieur (K) du tibia (T),
et les au moins deux géométries de référence (R1, R1', R2, R2') étant chacune adaptées pour le montage par complémentarité de forme au moins médiolatéral de la tige d'alignement extramédullaire (100, 100', 100"),
**caractérisé en ce que** la section de référence (3, 3a) présente au moins deux premières géométries de référence (R1, R1') qui sont espacées l'une de l'autre le long d'une première droite de référence (RG1) et deux deuxièmes géométries de référence (R2, R2') qui sont espacées l'une de l'autre le long d'une deuxième droite de référence (RG2), la première droite de référence (RG1) étant espacée de la droite d'appui (SG) d'une première distance antérieure (A1), et la deuxième droite de référence (RG2) étant espacée de la droite d'appui (SG) d'une deuxième distance antérieure (A2).

2. Dispositif chirurgical (1, 1a) selon la revendication 1, **caractérisé en ce que** les géométries de référence (R1, R1', R2, R2') sont chacune réalisées sous la forme d'un alésage (4, 4'), de telle sorte qu'il existe au moins deux premiers alésages (B1) et deux deuxièmes alésages (B2).

3. Dispositif chirurgical (1, 1a) selon la revendication 2, **caractérisé en ce que** les premiers alésages (B1) et les deuxièmes alésages (B2) sont reliés entre eux de manière antéropostérieure, moyennant quoi la tige d'alignement extramédullaire (100, 100', 100") peut être déplacée de manière antéropostérieure et/ou dans sa direction radiale à partir d'un montage au niveau des deux premiers alésages (B1) dans un montage au niveau des deux deuxièmes alésages (B2) et inversement.

4. Dispositif chirurgical (1, 1a) selon la revendication 2 ou 3, **caractérisé en ce que** la section de référence (3, 3a) est élastiquement déformable dans la zone des alésages (4, 4').

5. Dispositif chirurgical (1, 1a) selon l'une quelconque des revendications précédentes, **caractérisé en ce que** la section de référence (3, 3a) est réalisée sous forme d'étrier (30, 30a) avec deux branches longitudinales (31, 32, 31a, 32a) et une branche transversale (33, 33a), les deux branches longitudinales (31, 32, 31a, 32a) étant espacées proximodistalement l'une de l'autre et s'étendant chacune longitudinalement de manière antéropostérieure et présentant les géométries de référence (R1, R1', R2, R2'), et la branche transversale (33, 33a) s'étendant longitudinalement de manière proximodistale entre les deux branches longitudinales (31, 32, 31a, 32a) et les reliant entre elles.

6. Dispositif chirurgical (1, 1a) selon la revendication 5, **caractérisé en ce que** les branches longitudinales (31, 32, 31a, 32a) présentent chacune une rangée d'alésages (41, 41', 41a, 41a') avec une pluralité d'alésages (4, 4') en série de manière antéropostérieure en tant que géométries de référence.

7. Dispositif chirurgical (1a) selon la revendication 5 ou 6, **caractérisé en ce que** la section d'appui (2a) est formée par des extrémités frontales des branches longitudinales (31a, 32a), une extrémité frontale respective présentant l'une des géométries d'appui (Sa, Sa').

8. Dispositif chirurgical (1a) selon la revendication 7, **caractérisé en ce que** les géométries d'appui (Sa, Sa') présentent chacune une forme de fourche avec une première section de barre (311a, 321a) et une deuxième section de barre (312a, 322a).

9. Dispositif chirurgical (1) selon l'une quelconque des revendications 1 à 6, **caractérisé en ce que** la section d'appui (2) est réalisée sous forme de plaque (20) avec un côté inférieur (22) orienté postérieurement et un côté supérieur (21) orienté antérieurement, le côté inférieur (22) étant plan et présentant et/ou formant les au moins deux géométries d'appui (S, S'), et la section de référence (3) faisant saillie du côté supérieur (21).

10. Dispositif chirurgical (1) selon la revendication 9, **caractérisé en ce que** la plaque (20) présente une échelle (5) agencée sur le côté supérieur (21) avec des graduations (51) réparties distalement en éventail, les graduations (51) représentant chacune une valeur d'un angle varus/valgus du tibia (T).

11. Agencement (200, 200a) avec un dispositif chirurgical (1, 1a) selon l'une quelconque des revendications précédentes et avec une tige d'alignement extramédullaire (100, 100', 100").
